# EUROPÄISCHE PATENTSCHRIFT

(11) **EP 1 824 373 B1**
(45) Veröffentlichungstag und Bekanntmachung des Hinweises auf die Patenterteilung: **31.03.2010**
(21) Anmeldenummer: 05799999.7
(22) Anmeldetag: 31.10.2005
(51) Int. Cl.: A47L 15/42, A47L 15/00, A47L 15/46, A61L 2/24

(54) **VERFAHREN ZUR BEURTEILUNG UND SICHERSTELLUNG VON THERMISCHER HYGIENEWIRKUNG**
METHOD FOR EVALUATION AND GUARANTEEING THERMAL HYGIENIC EFFECTS
PROCEDE POUR EVALUER ET GARANTIR UN EFFET HYGIENIQUE THERMIQUE

(30) Priorität: 19.11.2004 DE 102004056052
(43) Veröffentlichungstag der Anmeldung: 29.08.2007
(73) Patentinhaber: MEIKO Maschinenbau GmbH & Co. KG, 77656 Offenburg (DE)
(72) Erfinder: MÄNNLE, Bruno, 77770 Durbach (DE); GAUS, Bruno, 77654 Offenburg (DE); LEHMANN, Denis, 77799 Ortenberg (DE)
(74) Vertreter: Hörschler, Wolfram Johannes
(86) Internationale Anmeldenummer: PCT/EP2005/011661
(87) Internationale Veröffentlichungsnummer: WO 2006/053634

(56) Entgegenhaltungen:
- EP-A- 0 727 179
- EP-A- 0 972 529
- EP-A- 0 978 267
- EP-A- 1 319 411
- DE-A1- 4 003 987
- DE-A1- 4 101 731
- FR-A- 2 558 713
- US-A1- 2001 033 805
- US-A1- 2003 168 080

## Beschreibung

### Technisches Gebiet

Die vorliegende Erfindung bezieht sich auf ein Verfahren zur Beurteilung und Sicherstellung der thermischen Hygienewirkung insbesondere einer Geschirrspülmaschine auf das zu reinigende Gut während des Programmablaufs in der Geschirrspülmaschine.

### Stand der Technik

Aus EP 1 371 319 B1 ist eine Geschirrspülmaschine mit in zwei Teile teilbarem Spülbehälter bekannt. Eine Geschirrspülvorrichtung umfasst ein Elektroventil, welches den Eintritt von Wasser aus einem Leitungsnetz steuert sowie eine Weichmachereinheit für Wasser sowie einen Spülbehälter, eine Abflussleitung und einen Mikroprozessor. Der Spülbehälter umfasst zwei Gestelle, welche Seite an Seite angeordnet sind sowie eine entfernbare zentrale Trennwand, welche die Teilung des Behälters in zwei unabhängige Sektoren bewirkt, von denen jeder mit einer Sammelwanne mit passendem Filter ausgestattet ist, von welcher das Wasser einer Spülpumpe und einer Abflusspumpe zufließt. Ferner sind ein Heizwiderstand, ein Spülmittelverteiler und wenigstens eine Sprinklervorrichtung vorgesehen. Die Wasserzufuhr von der Weichmachereinheit erfolgt über ein Deviationsventil, welches das Wasser zu den jeweiligen Zufuhrleitungen von Sektoren leitet. Der Abfluss erfolgt durch einen Verbinder, welcher die Abflussleitungen der beiden Sektoren in eine Abflussleitung zusammenführt und dadurch, dass ein Vierwegeverteiler vorgesehen ist, wobei der Verteiler Wasser von den Spülpumpen durch entsprechende Leitungen erhält. In diesen wird der Fluss durch jeweilige Ventile gesteuert, wobei der Verteiler ebenfalls die beiden Wannen durch entsprechende Leitungen, welche mittels eines Zentralventils des Verteilers getrennt sind, gegenseitig verbindet.

Aus EP 1 374 754 A1 ist eine Einbau-Geschirrspülmaschine mit erhöhter Ladekapazität bekannt. Eine Ladetür der Einbau-Geschirrspülmaschine wird durch eine Frontplatte gebildet, die von einem Rahmen umschlossen ist. Der Rahmen weist eine erhöhte Höhe auf und ist mit einer Anlagefläche versehen, welche tief gezogen ausgebildet ist und innerhalb einer Ausnehmung verläuft, so dass Raum eingespart werden kann.

Aus DE 101 56 559 C2 ist eine Einrichtung zur Temperatur-Messkontrolle im Spülbehälter einer Geschirrspülmaschine bekannt geworden. Die Geschirrspülmaschine umfasst ein oder mehrere Spülgut-Einsätze oder Geschirrkörbe mit einem türverschließbaren Spülbehälter. Der Spülbehälter ist mit einem aus dem Gerät herausgeführten separaten Sondenkanal für in den Behälter einschiebbare Mess-Sondenleitungen versehen. Der Sondenkanal mündet vorzugsweise von der Frontseite der Geschirrspülmaschine ausgehend an der Oberseite in den Spülbehälter. Der von der Oberseite her in den Spülbehälter einmündende Sondenkanal ist mit einer im oberen Gerätebereich zugänglich angeordneten Messöffnung versehen, wobei die Messöffnung im Gerätetürbereich dicht unterhalb des Gerätedeckels vorgesehen ist. Die Messöffnung kann auch im Bereich der oberen Türkante der Gerätetür des Spülbehälters ausgebildet sein. Der Sondenkanal ist als abgewinkelter Durchführungskanal für die Mess-Sondenleitungen ausgebildet und wenigstens an einem Ende verschließbar.

Ein abgewinkelt in den Spülbehälter an dessen Oberseite einmündende Kanalteil des Sondenkanals lässt sich mittels eines Gewindestopfens verschrauben; ferner kann der Sondenkanal im Deckenwandteil des Spülbehälters vorzugsweise lösbar fixiert werden.
Der Sondenkanal ist mit Gefälle zum Spülraum hin geneigt im Gerät ausgebildet. Der Sondenkanal ist hinsichtlich seines Querschnitts so bemessen, dass zur Temperatur-Messkontrolle im Spülbehälter mehrere Mess-Sondenleitungen durch ein und denselben Sondenkanal in den Spülbehälter eingeschoben werden können.

EP 1 319 411 A1 bezieht sich auf eine Reinigungs- und Desinfektionsmaschine. Diese umfasst mindestens einen Sensor, der eine physikalische Messgröße, wie z.B. Wassertemperatur, Menge eines zugegebenen Reinigungs- und Desinfektionsmittels, misst und an eine Steuereinheit meldet. Zur Messung ein und derselben physikalischen Größe werden zwei Sensoren verwendet, die je mit unterschiedlichen physikalischen Messprinzipien arbeiten. Zur Messung der Temperatur ist ein Sensor als Temperaturfühler ausgebildet und der andere Sensor ist ein Sensor, der der Erfassung der Heizleistung einer Heizeinrichtung dient. Die Lösung gemäß EP 1 319 411 A1 ermöglicht mithin eine redundante Messung von physikalischen Größen zur Erhöhung der Sicherheit der Validierung derselben.

Bei programmgesteuerten Geschirrspülmaschinen, insbesondere bei Spülautomaten für Labore und für Krankenhäuser, die mit temperaturabhängigen Programmen arbeiten, wie zum Beispiel Desinfektionsprogramme oder dergleichen und zur Aufbereitung von medizinischem Spülgut dienen, ist es ein gesetzliches Erfordernis, dass jeder Spülautomat, wie zum Beispiel ein Thermo-Desinfektor, in regelmäßigen Zeitintervallen auf Erreichen der notwendigen Desinfektionstemperatur kontrolliert wird. Hierzu sind mit einem Messsystem Temperaturmessungen an mehreren Stellen im Spülraum durchzuführen. Dabei werden je nach Anforderung bis zu 5 Sensoren durch eine spezielle Messöffnung in den Spülraum eingebracht. Die beispielsweise als NTC-Fühler und/oder PT100-Elemente ausgebildeten Sonden umfassen Mess-Sondenleitungen aus nicht isolierten Drähten von etwa 0,5 mm Durchmesser, die eine Länge von ca. 1 m aufweisen beziehungsweise isolierte Kabel. Zur Durchführung der Kontrollmessungen ist es bekannt, die Mess-Sondenleitungen durch den Türspalt zwischen Gerätetür und Spülraumdichtung oder Türdichtung zu verlegen. Eine derartige Leitungsverlegung ist jedoch bei dickeren Sensorkabeln mit Problemen behaftet. Weil eine Temperatur-Messkontrolle innerhalb des Spülbehälters nur bei geschlossener Gerätetür durchgeführt werden kann, wird die Türdichtung durch die geklemmten Leitungen teilweise so stark deformiert, dass Undichtigkeiten auftreten. Auch eine separate Messöffnung in der Gerätetür hätte den Nachteil, dass sich beim Schließen der Tür die freiliegenden Kabel unkontrolliert im Spülraum bewegen und gegebenenfalls sogar in den Drehbereich der korbzugeordneten Sprüharme gelangen und diese blockieren.

Die aus dem Stand der Technik bekannten Geschirrspülautomaten weisen üblicherweise mindestens eine Spülkammer und einen Spülwassertank auf. Eine Umwälzpumpe saugt aus dem Spülwassertank Spülwasser an und versprüht es über ein Spülsystem innerhalb der Spülkammer auf das schmutzige, zu reinigende Spülgut. Das versprühte Spülwasser fällt anschließend in den Spülwassertank zurück und wird erneut von der Umwälzpumpe angesaugt und auf das Spülgut versprüht.

Bei üblicherweise gewerblich genutzten Geschirrspülmaschinen wird nach der Vorreinigung des Spülguts durch das Spülwasser aus dem Spülwassertank entweder in einem weiteren Programmschritt heißes Nachspülwasser beziehungsweise Frischwasser mittels eines weiteren Sprühsystems über das zu reinigende Gut versprüht oder das verschmutzte Spülwasser durch heißes Frischwasser ersetzt und mit dem gleichen Spülsystem über das zu reinigende Spülgut versprüht. Die in Geschirrspülmaschinen nach dem Programmablauf erreichte Keimreduktion hängt dabei in erheblichem Maße von der Temperatur des Spülwassers beziehungsweise Nachspülwassers, der Spüldauer, der mechanischen Spülwirkung sowie der Reinigerkonzentration ab.

Zur Beurteilung der nach dem Spülprogrammablauf erreichten Hygienisierung des Spülgutes werden Testverfahren herangezogen, welche auf der Keimreduktion bestimmter Testkeime an der Spülgutoberfläche basieren.

Gemäß der DIN 10511-12 C.3 für Deutschland wird ein Verfahren beschrieben, bei dem mit definiert angeschmutzten Gläsern die Keimreduktion nach Programmablauf über sogenannte Abklatschuntersuchungen ermittelt wird. Hierbei muss im kürzesten beziehungsweise ungünstigen Spülprogramm eine Mindest-Keimreduktion erreicht werden. Als Testkeim beziehungsweise Organismus wird bei diesem Test E. faecium ATCC 6057 verwendet. Aus den Ergebnissen dieser Untersuchung ergeben sich dann Mindestanforderungen hinsichtlich Temperatur, Spüldauer, Reinigerkonzentration und mechanischer Spülleistungen, mit der dann diese Spülmaschine beziehungsweise diese Spülmaschinen-Baureihe werksseitig voreingestellt werden muss, um im Betrieb beim Kunden die geforderte Keimreduktion zu erreichen. Von Nachteil an diesem Verfahren ist der Umstand, dass diese Prüfverfahren vor Ort beim Kunden nur mit sehr viel Aufwand durchgeführt werden kann und sich somit gerade bei kleinen Spülmaschinen nicht zur Überprüfung der einwandfreien Funktion der Spülmaschine eignet.

Aus den USA ist ein Verfahren bekannt, welches im NSF-Standard 3 beschrieben wird. Basis dieses Verfahrens ist die aus Versuchen ermittelte Keimreduktion von Tuberkulosebakterien durch die Einwirkung einer Temperatur über die Zeit. Das Einwirken der Temperatur über die Zeit wird als "Wärmeäquivalent" (HUE) bezeichnet. Wie viele Wärmeäquivalente pro Sekunde bei welcher Temperatur erzielt werden, ist in einer Tabelle, die diesem Verfahren zugrundegelegt wird, niedergeschrieben. Anhand von Versuchen wurden für Spülmaschinen eine Mindesttanktemperatur und eine Mindestnachspültemperatur definiert, welche die Spülmaschine erreichen muss, um die geforderte Keimreduktion zu erreichen. Für den Hersteller von Spülmaschinen bedeutet dies, dass diese Temperaturen werksseitig fest in der Spülmaschinensteuerung der Geschirrspülmaschine voreingestellt werden müssen. Bei der Verfahrensprüfung einer Spülmaschine nach diesem Verfahren wird ein Temperatursensor auf einem Teller angebracht. Anschließend wird dieser Teller in einer vorgegebenen Position im Geschirrkorb der Geschirrspülmaschine platziert und die Temperatur während des Programmablaufs aufgezeichnet. Aus dem Temperaturverlauf während des gesamten Spülprogramms werden dann anhand der oben erwähnten Tabelle die auf dem Teller über den gesamten Programmablauf einwirkenden Wärmeäquivalente ermittelt. Diese Prüfung ist für drei verschiedene Tellerpositionen innerhalb des Geschirrkorbs vorgeschrieben. Zur Erfüllung der geforderten Keimreduzierung müssen nach der Vorschrift NSF-3 mindestens 3600 Wärmeäquivalente in jeder Tellerposition innerhalb des Geschirrkorbs erreicht werden. Vorteilhaft an diesem Verfahren ist, dass dieses Verfahren mit relativ wenig Aufwand vor Ort beim Kunden durchgeführt werden kann, um die einwandfreie Funktion der Spülmaschine zu überprüfen.

Im europäischen Bereich gilt für Reinigungs-/Desinfektionsgeräte die prEN ISO 15883-1, welche zur Beurteilung der Hygienewirkung ebenfalls den Zusammenhang zwischen der Keimreduzierung und der Temperatur über die Zeit heranzieht. Dieser Zusammenhang wird als A₀-Wert bezeichnet und ist ebenfalls in einer Tabelle niedergeschrieben beziehungsweise errechnet sich gemäß einer mathematischen Beziehung. Der A₀-Wert ist definiert als das Zeitäquivalent in Sekunden bei einer Temperatur von 80 °C, bei dem eine gegebene Desinfektionswirkung ausgeübt wird und entspricht sinngemäß den Wärmeäquivalenten (HUE) gemäß des NSF-Standards 3. Auch hierbei ist ein mindestens zu erreichender A₀-Wert an jeder Stelle in der Spülkammer des Reinigungs- und Desinfektionsgerätes sicherzustellen. Dies bezieht sich demnach auch auf die ungünstigste Stelle bei dem ungünstigsten Programm. Dieses Verfahren wird derzeit nicht zur Beurteilung der Hygienewirkung gewerblicher Spülmaschinen im europäischen Raum herangezogen.

Allen oben skizzierten Verfahren ist gemeinsam, dass stets das ungünstigste Spülprogramm beziehungsweise die ungünstigsten Bedingungen zur Beurteilung der Keimreduktion herangezogen werden. Nachteilig an diesem Vorgehen ist jedoch der Umstand, dass sich durch die starren Vorgaben für die Spülprogramme, die sich hinsichtlich Temperatur oder weiterer Programmparameter von diesen Vorgaben unterscheiden, unter Umständen unnötig lange Programmlaufzeiten ergeben, was höchst unerwünscht ist. So könnte zum Beispiel durch eine Erhöhung der Wasch- und/oder Nachspültemperatur die Programmlaufzeit bei gleichbleibender Keimreduktion verkürzt werden.

Angesichts der oben skizzierten Nachteile der bisher gebräuchlichen Verfahren zur Bestimmung der Hygienewirkung an Geschirrspülautomaten liegt der vorliegenden Erfindung die Aufgabe zugrunde, ein Verfahren bereitzustellen, welches die Nachteile der oben aus dem Stand der Technik bekannten Verfahren umgeht und gleichzeitig die Prozesssicherheit hinsichtlich der Sicherstellung und dauerhaften Gewährleistung der thermischen Hygienewirkung innerhalb eines Geschirrspülautomaten erlaubt.

### Darstellung der Erfindung

Erfindungsgemäß wird vorgeschlagen, in der Spülkammer der Spülmaschine einen Temperatursensor vorzusehen, welcher die in der Spülkammer herrschende Temperatur während des gesamten Programmablaufs des Geschirrspülautomaten erfasst. Der Temperatursensor ist fester Bestandteil der Geschirrspülmaschine und demzufolge fest in der Geschirrspülmaschine eingebaut. Der Temperatursensor wird derart mit dem innerhalb eines NSF-Standard 3-Tests oder innerhalb des A₀-Tests eingesetzten Temperaturfühlers abgeglichen, dass über eine Steuerung, die innerhalb oder außerhalb der Geschirrspülmaschine angeordnet ist, die gleichen Wärmeäquivalente wie in den NSF- 3 Standard-Testverfahren beziehungsweise in dem A₀-Testverfahren erfasst werden können. Über den innerhalb der Spülkammer der Geschirrspülmaschine angeordneten Temperatursensor und die mit diesem verbundene Steuerung lassen sich die momentan erreichten Wärmeäquivalente innerhalb der Spülkammer des Geschirrspülautomaten ermitteln. In der innerhalb des Geschirrspülautomaten oder extern am Geschirrspülautomaten vorgesehenen Steuerung, mit welcher der Temperatursensor verbunden ist, können die Tabellenwerte des NSF3-Standards beziehungsweise des A₀-Testverfahrens hinterlegt werden und mit den über den innerhalb der Spülkammer angeordneten Temperatursensoren ermittelten Werte verglichen werden.

Ist ein in der Steuerung der Geschirrspülmaschine beziehungsweise einer extern vorgesehenen Steuerung voreingestellter Wert für die Wärmeäquivalente erreicht, kann zum Beispiel über ein optisches oder ein akustisches Signal das Ende des Programmablaufes angezeigt werden. Dies wird dann beendet, so dass eine höchst unerwünschte Programmlaufzeitverlängerung unterbleiben kann. Dies schont die Ressourcen und setzt darüber hinaus den Energieverbrauch des Geschirrspülautomaten während des Betriebes bei jedem Programmablauf erheblich herab.

In vorteilhafter Ausgestaltung des der Erfindung zugrunde liegenden Gedankens kann das Erreichen des Endes des Programmablaufes beispielsweise mit der Entriegelung der Gerätetür der Geschirrspülmaschine gekoppelt werden. Nach dem Erreichen der über den Temperatursensor erfassten, jeweils geforderten Wärmeäquivalente kann der Verriegelungsmechanismus der Gerätetür aktiviert werden und die Gerätetür entriegeln. Somit ist sichergestellt, dass das zu reinigende und gegebenenfalls zu desinfizierende Spülgut nicht vor Erreichen der erforderlichen Wärmeäquivalente innerhalb der Spülkammer aus der Geschirrspülmaschine entnommen werden kann.

Durch das erfindungsgemäß vorgeschlagene Verfahren können bei Programmabläufen im Programmschritt "Spülen" und/oder "Nachspülen" das Spülprogramm nach Erreichen der voreingestellten Wärmeäquivalente, die durch die intern oder extern vorgesehene Steuerung des Temperatursensors vorgegeben werden und unter der Voraussetzung, dass der Nachspülschritt ausgeführt ist, rechtzeitig beendet werden und eine überflüssige weitere Programmlaufzeitverlängerung unterbunden wird.

Ein weiterer Vorteil der erfindungsgemäß vorgeschlagenen Lösung ist darin zu erblicken, dass die Geschirrspülmaschine aktiv die Hygiene, das heißt den Desinfektionsgrad des zu reinigenden Spülgutes überwacht und Unregelmäßigkeiten im Spülbetrieb, wie zum Beispiel das Einbringen von einer größeren Menge kalten Wassers durch geeignete Maßnahmen automatisch korrigieren kann. Eine geeignete Maßnahme, die bei Einbringen einer größeren Menge kalten Wassers in die Spülkammer ergriffen werden kann, besteht zum Beispiel darin, die Laufzeit des Nachspülschrittes zu verlängern, bis die Signalisierung des Programmendes entweder auf optischem oder auf akustischem oder auch durch ein Entriegeln der Gerätetür angezeigt wird.

Durch das erfindungsgemäß vorgeschlagene Verfahren, bei dem die Werte für die zu erreichenden Wärmeäquivalenten gemäß NSF3-Standard und gemäß A₀-Wertverfahren in einer extern oder intern an der Geschirrspülmaschine vorgesehenen Steuerung implementiert sind, lässt sich sicherstellen, dass immer eine den Vorgaben entsprechende thermische Hygienewirkung unter der optimalen beziehungsweise kürzesten Programmlaufzeit erreicht wird. Durch das erfindungsgemäß vorgeschlagene Verfahren, bei dem die Werte für die Äquivalente in der Steuerung implementiert sind, können die während des Programmablaufs erreichten Wärmeäquivalenzwerte über ein Display, welches von der Steuerung angesteuert wird, angezeigt werden. Dadurch ist es dem Betreiber möglich, zu jedem Zeitpunkt des Programmablaufs den jeweiligen Stand der für die thermische Hygienewirkung erreichten Wärmeäquivalente abzulesen und den Programmfortschritt zu beurteilen.

Durch das erfindungsgemäß vorgeschlagene Verfahren können auch in einem Krankenhaus oder Pflegeheim eingesetzte Reinigungs- und Desinfektionsautomaten (Steckbeckenspüler), in denen Steckbecken, Urinflaschen sowie andere Auffangbehältnisse für menschliche Ausscheidungen gereinigt und desinfiziert werden, betrieben werden. Die Desinfektion dieser Behältnisse kann sowohl thermisch, d.h. mit Dampf, als auch chemisch erfolgen. Mit dem erfindungsgemäß vorgeschlagenen Verfahren lässt sich während des Betriebes solcher Reinigungs- und Desinfektionsautomaten, die mit Dampf desinfizieren, deren thermische Hygienewirkung auf die zu reinigenden Behältnisse beurteilen. Dazu wird die Reinigungskammer dieser Automaten ebenfalls mit einem Temperatursensor versehen, welcher die in der Reinigungskammer herrschende Temperatur während des gesamten Programmablaufs innerhalb des Reinigungs- und Desinfektionsautomaten erfasst. Der Temperatursensor, der in der Reinigungskammer des Reinigungs- und Desinfektionsautomaten vorgesehen ist, wird mit einem innerhalb eines NSF-Standard 3-Tests oder innerhalb eines A₀-Tests eingesetzten Temperaturfühlers abgeglichen, so dass über die Steuerung des Reinigungs- und Desinfektionsautomaten die momentan jeweils erreichte Temperatur und Einwirkzeit auf das zu reinigende Gut erfasst und daraus die entsprechenden Wärmeäquivalente berechnet werden können.

### Zeichnung

Anhand der Zeichnung wird das erfindungsgemäß vorgeschlagene Verfahren anhand eines Einkammer-Geschirrspülautomaten und anhand eines Reinigungs- und Desinfektionsautomaten näher erläutert.

Es zeigt:
- Figur 1: eine Einkammer-Geschirrspülmaschine in schematischer Wiedergabe und
- Figur 2: einen Reinigungs- und Desinfektionsautomaten in schematischer Darstellung.

### Ausführungsvarianten

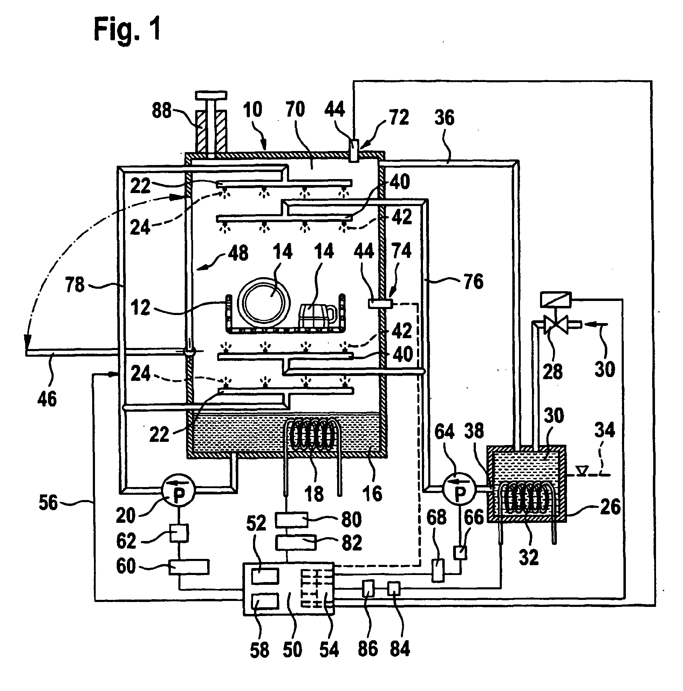

Figur 1 ist eine Geschirrspülmaschine zu entnehmen, bei der es sich um eine Einkammer-Geschirrspülmaschine handelt, die über eine Gerätetür mit zu reinigendem Spülgut beschickt werden kann.

Eine Einkammer-Spülmaschine 10 umfasst eine Spülkammer 70, in der ein Korb 12 aufgenommen ist. Innerhalb des Korbes 12 befindet sich Spülgut 14, bei dem es sich um Teller, Tassen, Besteck, Gläser, Kochgeschirr etc. handeln kann. Im unteren Bereich der Einkammer-Spülmaschine 10 befindet sich ein Tank 16 zur Aufnahme der mit Reiniger versetzten Spüllauge. Die Spüllauge, die im Tank 16 enthalten ist, wird über ein Heizelement 18 erwärmt. Vom Tank 16 der Einkammer-Spülmaschine 10 wird die Spüllauge über eine Umwälzpumpe 20 zu einem Spülsystem 22 für Spüllauge transportiert. Das Spülsystem 22 für Spüllauge steht mit der Umwälzpumpe 20 über eine Spüllaugenleitung 78 in Verbindung. Im Spülsystem 22 für Spüllauge, bei welchem es sich bevorzugt um Düsen 24 aufweisende Dreharme handelt, ist sowohl im oberen Bereich der Spülkammer 70 als auch in deren unteren Bereich angeordnet.

Der Einkammer-Geschirrspülmaschine 10 ist darüber hinaus ein Frischwasser-Boiler 26 zugeordnet. Dem Frischwasser-Boiler 26 wiederum ist ein Füllventil 28 vorgeschaltet, über welches der Zulauf von Frischwasser 30 in den Frischwasser-Boiler 26 gesteuert werden kann. Das Füllventil 28 steht mit einer Steuerung 50 der Einkammer-Geschirrspülmaschine in Verbindung, welche ihrerseits den Programmablauf der einzelnen in der Einkammer-Geschirrspülmaschine 10 ablaufenden Programmschritte steuert. Innerhalb des Frischwasser-Boilers 26 befindet sich ein Heizelement 32, mit welchem das in den Frischwasser-Boiler 26 nachströmende Frischwasser 30 sowie das sich innerhalb des Frischwasser-Boilers 26 befindende Frischwasser 30 erwärmt werden. Der Pegelstand des Frischwassers 30 innerhalb des Frischwasser-Boilers 26 ist durch Bezugszeichen 34 bezeichnet und liegt stets so, dass das Heizelement 32 innerhalb des Frischwasser-Boilers 26 mit Frischwasser 30 bedeckt ist. Der Frischwasser-Boiler 26 und die Spülkammer 70 der Einkammer-Geschirrspülmaschine 10 stehen über eine Entlüftungsleitung 36 in Verbindung. Am Frischwasser-Boiler 26 ist eine Ansaugstelle 38 vorgesehen, über welche eine Frischwasserpumpe 64 erwärmtes Frischwasser 30 über eine Frischwasserleitung 76 an Spülsysteme 40 für Frischwasser fördert. Bei den Spülsystemen 40 für Frischwasser handelt es sich ebenfalls bevorzugt um Dreharme, an deren dem Korb 12 zuweisenden Seiten Düsen 42 ausgebildet sind, über welche das erwärmte Frischwasser 30 auf das zu reinigende Spülgut 14 aufgegeben wird.

In der Spülkammer 70 der Einkammer-Geschirrspülmaschine 10 befindet sich ein Temperatursensor 44, der entweder in einer ersten Temperatursensorposition 72 an der Decke der Spülkammer 70 angeordnet sein kann oder in einer zweiten Temperatursensorposition 74 an der Rückwand der Spülkammer 70 der Einkammer-Geschirrspülmaschine 10 aufgenommen sein kann. Die Einbauposition 72, 74 des Temperatursensors 44 ist beispielhaft dargestellt, je nach den sonstigen Rahmenbedingungen wird die Position des Temperatursensors 44 so gewählt, dass die ermittelte Temperatur am Temperatursensor 44 während des Programmablaufs vergleichbar mit dem Temperaturverlauf des Spülgutes oder des Reinigungsgutes ist.

Der Temperatursensor 44 ist mit der Steuerung 50 der Einkammer-Geschirrspülmaschine 10 verbunden. Die Spülkammer 70 der Einkammer-Geschirrspülmaschine 10 ist über eine Gerätetür 46 zugänglich, welche eine Ladeöffnung 48 freigibt oder verschließt. Der Gerätetür 46 ist ein Verriegelungsmechanismus zugeordnet, über welchen die Gerätetür 46 in ihrer Schließstellung verriegelt wird. Der Verriegelungsmechanismus für die Gerätetür 46 ist ebenfalls mit der Steuerung 50 der Einkammer-Geschirrspülmaschine 10 verbunden.

Die Steuerung 50, die der Einkammer-Geschirrspülmaschine 10 zugeordnet ist, kann entweder eine interne, das heißt innerhalb der Einkammer-Geschirrspülmaschine 10 angeordnete Steuerung sein, oder auch eine externe, das heißt außerhalb der Einkammer-Geschirrspülmaschine 10 untergebrachte Steuerung sein. Die Steuerung 50 umfasst einen Mikroprozessor (CPU) 52 sowie einen Datenspeicher 54. Über eine Hauptsteuerleitung 56 erfolgt die Steuerung sämtlicher Funktionen hinsichtlich der in der Einkammer-Geschirrspülmaschine 10 ablaufenden Programmschritte, das heißt auch des innerhalb der Einkammer-Geschirrspülmaschine 10 ablaufenden Verfahrens zur Beurteilung der Hygienewirkung. Die Steuerung 50 umfasst darüber hinaus eine Messdatenerfassungseinheit 58, über welche die von dem mindestens einen Temperatursensor 44, der der Spülkammer 70 zugeordnet ist, erfassten Messwerte gespeichert werden.

Darüber hinaus steuert die Steuerung 50 über einen Leistungsregler 60, der der Umwälzpumpe 20 vorgeschaltet ist, deren elektrische Energieversorgung 62. Auch der Frischwasserpumpe 64 ist ein Leistungsregler 68 vorgeschaltet, über den die elektrische Energieversorgung 66 der Frischwasserpumpe 64 gesteuert werden kann. Gleiches gilt für einen Leistungsregler 82, über welchen die Energieversorgung 80 des Heizelementes 18 für die Spüllauge vorgeschaltet ist sowie für einen Leistungsregler 86, der die Energieversorgung 84 des Heizelementes 32 des Frischwasser-Boilers 26 steuert.

In der Steuerung 50 sind innerhalb des dort vorgesehenen Datenspeichers 54 die Werte für die Wärmeäquivalente abgespeichert, die entweder gemäß des NSF3-Standards oder gemäß des A₀-Wertverfahrens maßgeblich sind, um die Hygienewirkung eines Geschirrspülautomaten zu bestimmen beziehungsweise zu klassifizieren. Im Datenspeicher 54 der Steuerung 50 lassen sich zum Beispiel die der nachfolgend wiedergegebenen A₀-Werte gemäß der in Europa zu erwartenden EN-ISO 15883 abspeichern:

| **Temperatur** | **A₀ 60 (sec)** | **A₀ 600 (sec)** | **A₀ 3.000** | |
|---|---|---|---|---|
| | | | **(sec)** | **=(min)** |
| 65° | 1.897,4 | 18.973,7 | 94.868,3 | 1.581,1 |
| 66° | 1.507,1 | 15.071,3 | 75.356,6 | 1.255,9 |
| 67° | 1.197,2 | 11.971,6 | 59.857,9 | 997,6 |
| 68° | 950,9 | 9.509,4 | 47.546,8 | 792,4 |
| 69° | 755,4 | 7.553,6 | 37.767,8 | 629,5 |
| 70° | 600,0 | 6.000,0 | 30.000,0 | 500,0 |
| 71° | 476,6 | 4.766,0 | 23.829,8 | 397,2 |
| 72° | 378,6 | 3.785,7 | 18.928,7 | 315,5 |
| 73° | 300,7 | 3.007,1 | 15.035,6 | 250,6 |
| 74° | 238,9 | 2.388,6 | 11.943,2 | 199,1 |
| 75° | 189,7 | 1.897,4 | 9.486,8 | 158,1 |
| 76° | 150,7 | 1.507,1 | 7.535,7 | 125,6 |
| 77° | 119,7 | 1.197,2 | 5.985,8 | 99,8 |
| 78° | 95,1 | 950,9 | 4.754,7 | 79,2 |
| 79° | 75,5 | 755,4 | 3.776,8 | 62,9 |
| 80° | 60,0 | 600,0 | 3.000,0 | 50,0 |
| 81° | 47,7 | 476,6 | 2.383,0 | 39,7 |
| 82° | 37,9 | 378,6 | 1.892,9 | 31,5 |
| 83° | 30,1 | 300,7 | 1.503,6 | 25,1 |
| 84° | 23,9 | 238,9 | 1.194,3 | 19,9 |
| 85° | 19,0 | 189,7 | 948,7 | 15,8 |
| 86° | 15,1 | 150,7 | 753,6 | 12,6 |
| 87° | 12,0 | 119,7 | 598,6 | 10,0 |
| 88° | 9,5 | 95,1 | 475,5 | 7,9 |
| 89° | 7,6 | 75,5 | 377,7 | 6,3 |
| 90° | 6,0 | 60,0 | 300,0 | 5,0 |
| 91° | 4,8 | 47,7 | 238,3 | 4,0 |
| 92° | 3,8 | 37,9 | 189,3 | 3,2 |
| 93° | 3,0 | 30,1 | 150,4 | 2,5 |
| 94° | 2,4 | 23,9 | 119,4 | 2,0 |
| 95° | 1,9 | 19,0 | 94,9 | 1,6 |

Der der oben stehenden Tabelle entnehmbare A₀-Wert ist definiert als ein Zeitäquivalent in Sekunden, bei dem eine Desinfektionswirkung ausgeübt wird.

Der A₀-Wert eines Desinfektionsverfahrens mit feuchter Hitze kennzeichnet die Abtötung von Keimen, angegeben als Zeitäquivalent in Sekunden bei einer durch das Verfahren an das Produkt übertragenen Temperatur.

Der Temperatursensor 44, der in der Spülkammer 70 der Einkammer-Geschirrspülmaschine 10 eingesetzt wird, sei es in der ersten Temperatursensorposition 72 oder alternativ in der zweiten Temperatursensorposition 74 wird auf den innerhalb des NSF3-Standard-Testverfahrens oder den innerhalb des A₀-Testverfahrens verwendeten Temperaturfühler derart abgeglichen, dass über die Steuerung 50 innerhalb der Einkammer-Geschirrspülmaschine 10 die gleichen Wärmeäquivalente wie im NSF3-Standardverfahren beziehungsweise im A₀-Testverfahren ermittelt werden können. Über den innerhalb der Spülkammer 70 angebrachten Temperatursensor 44 und die Steuerung 50 werden die momentan innerhalb eines Programmablaufes erreichten Wärmeäquivalente ermittelt und mit den im Datenspeicher 54 abgelegten Tabellenwerten, zum Beispiel im Falle der EN ISO 15883 der dort hinterlegten Werte, verglichen. Sind die über den Temperatursensor 44 ermittelten Werte für die Wärmeäquivalente, die innerhalb der Spülkammer 70 des Einkammer-Geschirrspülmaschine 10 herrschen, zu niedrig, so kann über die Steuerung 50 entweder die Temperatur der Spüllauge, die im Spüllaugentank 16 vorhanden ist, erhöht werden, oder über die Steuerung 50 die Temperatur des Frischwassers 30, welches der Spülkammer 70 über die Spülsysteme 40 zugeführt wird, erhöht werden. Dazu werden über die Steuerung 50 die entsprechenden, der Umwälzpumpe 20 beziehungsweise der Frischwasserpumpe 64 zugeordneten Leistungsregler 60 beziehungsweise 68 angesteuert.

Werden die gemäß des NSF3-Standards oder die gemäß der EN-ISO 15883 vorgegebenen Werte für die in die Spülkammer 70 der Einkammer-Geschirrspülmaschine 10 eingetragenen Wärmeäquivalente erreicht, so wird der in der Spülkammer 70 der Einkammer-Geschirrspülmaschine 10 ablaufende Programmschritt beendet. Das Ende des jeweiligen, in der Spülkammer 70 ablaufenden Programmschrittes kann durch die Steuerung 50 entweder auf optischem Wege, zum Beispiel durch ein Aufleuchten des Displays, oder auch auf akustischem Wege angezeigt werden. Darüber hinaus besteht die Möglichkeit, den der Gerätetür 46 der Einkammer-Geschirrspülmaschine 10 zugeordneten Verriegelungsmechanismus nach Erreichen der vorgeschriebenen Wärmeäquivalente innerhalb der Spülkammer 70 über die Steuerung 50 zu entriegeln. In diesem Falle ist sichergestellt, dass das Spülgut 14 nicht vor Erreichen der erforderlichen Wärmeäquivalente aus der Spülkammer 70 entnommen wird, das heißt eine Entnahme von nur unzureichend thermisch behandeltem Spülgut 14 aus der Spülkammer 70 kann verhindert werden.

Durch das erfindungsgemäß vorgeschlagene Verfahren zur Beurteilung der Hygienewirkung der Einkammer-Geschirrspülmaschine 10 können bei Programmabläufen mit erhöhter Spülflüssigkeitstemperatur im Programmschritt "Spülen" und/oder "Nachspülen", die jeweiligen Spülprogramme nach Erreichen der voreingestellten Werte für die Wärmeäquivalente beendet werden, vorausgesetzt, dass der Nachspülschritt bereits ausgeführt ist

Ein weiterer Vorteil, der mit dem erfindungsgemäß vorgeschlagenen, an einer Einkammer-Geschirrspülmaschine 10 implementierten Verfahren zur Beurteilung der Hygienewirkung erzielbar ist, ist darin zu erblicken, dass die Geschirrspülmaschine aktiv die Hygiene des zu reinigenden Spülgutes 14 überwacht. Unregelmäßigkeiten im Spülbetrieb, wie zum Beispiel das Einbringen von einer größeren Menge kalten Wassers in den Tank für Spüllauge 16 und eine dadurch absinkende Temperatur in der Spülkammer 70, können durch geeignete über die Steuerung 50 einzuleitende Gegenmaßnahmen ausgeglichen werden. So kann zum Beispiel über die Steuerung 50 der Nachspülvorgang verlängert oder über eine entsprechende Ansteuerung des Leistungsreglers 82 für das Heizelement 18 des Tanks für Spüllauge 16 die Temperatur der Spüllauge entsprechend erhöht werden, um dem Kaltwassereintrag entgegenzuwirken und ein Absinken der in der Spülkammer 70 der Einkammer-Geschirrspülmaschine 10 herrschenden Temperatur zu verhindern.

Über das erfindungsgemäß vorgeschlagene Verfahren ist es nunmehr möglich, bei jedem Programmablauf unabhängig von Unregelmäßigkeiten, wie zum Beispiel durch unterschiedliches Spülgut, hinsichtlich Masse oder Temperatur des Spülgutes vor Beginn des Spülprogramms, die jeweiligen Wärmeäquivalente zu erfassen und gemäß den Vorgaben aus der NSF 3 Vorschrift oder der Norm EN ISO 15883-1 auszuwerten und den Programmablauf optimal zu steuern. Über ein Display können die erreichten Wärmeäquivalentwerte angezeigt werden. Dem Nutzer des Gerätes ist somit die Möglichkeit in die Hand gegeben, die thermische Hygienewirkung eines Einkammer-Geschirrspülautomaten während jedes Programmablaufs zu verfolgen beziehungsweise zu kontrollieren.

Mit Bezugszeichen 88 ist eine zum Beispiel als Elektromagnet ausbildbare Verriegelungseinrichtung gekennzeichnet, die mit der Steuerung 50 gekoppelt sein kann. Nach Erreichen der vorgeschriebenen Wärmeäquivalente innerhalb der Spülkammer 70 der Einkammer-Geschirrspülmaschine 10 wird diese Verriegelungseinrichtung 88 über die Steuerung 50 entriegelt. Dadurch ist sichergestellt, dass nur solches Spülgut 14 der Spülkammer 70 entnommen werden kann, welches der entsprechend den jeweiligen Standards vorgeschriebenen Wärmeäquivalenten ausgesetzt war, d.h. welches nunmehr der Spülkammer hygienisch gereinigt entnommen werden kann.

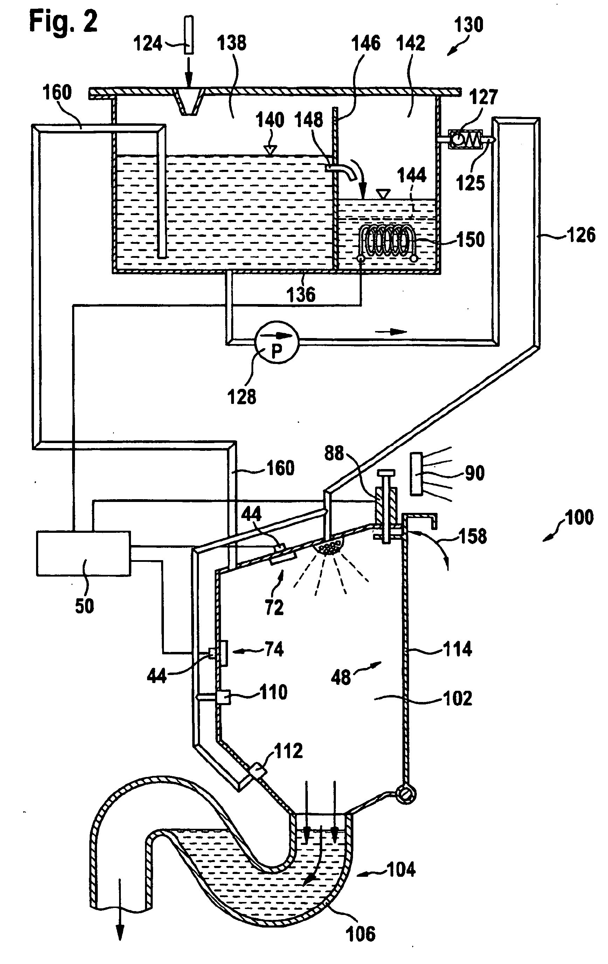

Figur 2 zeigt in schematischer Darstellung einen Reinigungs- und Desinfektionsautomaten, an welchem das erfindungsgemäß vorgeschlagene Verfahren zur Beurteilung und Überwachung der Hygienewirkung von Reinigungsgut ebenfalls implementiert werden kann.

Figur 2 ist ein Reinigungs- und Desinfektionsautomat 100 zu entnehmen, wie er in einem Krankenhaus oder Pflegeheimen zur Reinigung von Steckbecken, Urinflaschen sowie anderer Auffangbehältnisse für menschliche Ausscheidungen eingesetzt wird. Die Desinfektion innerhalb des Reinigungs- und Desinfektionsautomaten 100 kann sowohl thermisch, d.h. über Dampf als auch chemisch erfolgen. Dazu ist in dem Reinigungs- und Desinfektionsautomaten 100 in der Regel eine Reinigungskammer 102 ausgebildet, die von außen her über eine mittels der Verriegelungseinrichtung 88 verriegelbare Tür 114 mit zu reinigenden Behältnissen beladen werden kann und durch welche die gereinigten und desinfizierten Behältnisse nach erfolgter Reinigung und Desinfektion aus der Reinigungskammer 102 auch wieder entnommen werden können.

Die Reinigungskammer 102 umfasst an ihrem unteren Ende einen einen Siphonbogen 106 enthaltenen Ablauf 104, über welchen die Reste menschlicher Ausscheidungen in ein Abwassersystem eingeleitet werden können. Zur Vermeidung von Geruchsbildung in der Reinigungskammer 102 dient der im Ablauf 104 ausgebildete Siphonbogen 106, der im Allgemeinen aufgrund des sich darin ansammelnden Wassers als Geruchssperre in Abwassersystemen vorgesehen ist, so auch an dem hier beschriebenen Reinigungs- und Desinfektionsautomaten 100. Die Reinigungskammer 102 des Reinigungs- und Desinfektionsautomaten 100 ist durch eine schwenkbare Tür 114 zugänglich. Die Tür 114 ist um ein am unteren Ende der Tür 114 angebrachtes Scharnier bewegbar und lässt sich in Öffnungs-/Schließrichtung 158 gemäß des in der Zeichnung eingetragenen Doppelpfeils bewegen. Die Reinigungskammer 102 ist über eine Wasser-/Dampf-Einheit 130 mit Dampf beaufschlagbar. In Figur 2 sind Düsen 108, 110, 112 dargestellt, aus welchen Wasserdampf in die Reinigungskammer 102 eintreten kann. Diese sind in die Dachfläche der Reinigungskammer 102 integriert, können jedoch auch in deren seitlichen Begrenzungsflächen vorgesehen sein. Die Düsen 108, 110, 112, über welche Dampf in die Reinigungskammer 102 einleitbar ist, könnten ebensogut an der rückwärtigen Begrenzungswand der Reinigungskammer 102 angebracht sein. In die Reinigungskammer 102 mündet ferner ein Sicherheitsüberlauf 160, über welchen Wasser aus der Wasser- /Dampf-Einheit 130 im oberen Bereich des Reinigungs- und Desinfektionsautomaten 100 in die Reinigungskammer 102 überströmen kann und von dort in den Ablauf 104 gelangt. Der Sicherheitsüberlauf 160 könnte auch direkt in den Ablauf 104 münden.

Im oberen Bereich des Reinigungs- und Desinfektionsautomaten 100 gemäß der Darstellung in Figur 2 ist die Wasser-/Dampf-Einheit 130 aufgenommen. Der Wasser- /Dampf-Einheit 130 ist eine Wasserpumpe 128 zugeordnet, die unterhalb des Bodens 136 der Wasser-/Dampf-Einheit 130 angeordnet ist. Über die Wasserpumpe 128, die eine Druckerhöhung des Wassers herbeiführt, wird Wasser aus einem Wasserbehälter 138 der Wasser-/Dampf-Einheit 130 über einen Wasserzulauf 126 in die Reinigungskammer 102 gepumpt. Der Wasserbehälter 138 der Wasser-/Dampf-Einheit 130 wird über einen Kaltwasser- oder einen Warmwasserzulauf 124 versorgt. Je nach gebäudeseitigem Anschluss kann über den Wasserzulauf 124 entweder Kaltwasser in einem Temperaturbereich zwischen 10 °C und 30 °C in den Wasserbehälter 138 strömen oder, falls es sich um einen gebäudeseitigen Warmwasseranschluss handelt, Wasser mit einer Temperatur zwischen 45 °C und 60 °C in den Wasserbehälter 138 der Wasser-/Dampf-Einheit 130 einströmen. Auch eine Mischwasserbefüllung des Wasserbehälters 138 ist möglich. Das über den Wasserzulauf 124 zuströmende Warmwasser oder Kaltwasser strömt in die Wasser-/Dampf-Einheit 130 ein und befüllt diese bis zu einem durch Bezugszeichen 140 gekennzeichneten Pegelstand. Im Falle eines übermäßigen Wassereinlaufs strömt Wasser über einen Sicherheitsüberlauf 160 in die Reinigungskammer 102 ein und strömt über den stets geöffneten Ablauf 104 in das Abwassersystem ab, so dass keine Wasserschäden in dem Raum auftreten können, in dem der Reinigungs- und Desinfektionsautomat 100 aufgestellt ist.

Der Wasserbehälter 138 ist über eine Trennwand 146 von einem Dampferzeuger 142 der Wasser-/Dampf-Einheit 130 getrennt. Zur Befüllung des Dampferzeugers 142 mit Wasser erstreckt sich durch die Trennwand 146 eine Überströmleitung 148. Über die Überströmleitung 148 strömt Wasser in den Dampferzeuger 142 über. Nach dem Abpumpen stellt sich ein Pegel 144 im Dampferzeuger 142 ein. Zur Erwärmung des im Dampferzeuger 142 vorhandenen Wassers wird der im Dampferzeuger 142 enthaltene Wasservorrat über eine in der Zeichnung durch eine Wendel angedeutete Heizeinrichtung 150 erhitzt. Der bei der Erhitzung des Wassers entstehende Dampf wird über einen Leitungsabschnitt 125 in die sich von der Wasserpumpe 128 aus erstreckende Zulaufleitung 126 zur Reinigungskammer 102 geführt. Der Leitungsabschnitt 125 zwischen dem Dampferzeuger 142 und der Zulaufleitung 126 zur Reinigungskammer 102 ist durch ein Rückschlagventil 127 geschlossen. Aufgrund des Druckes des Wasserdampfes im Dampferzeuger 142 öffnet das Rückschlagventil 127, so dass Wasserdampf in die Zulaufleitung 126 zur Reinigungskammer 102 strömen kann. Über die Wasserpumpe 128 kann Wasser aus dem Wasserbehälter 138 auch über die Düsen 108 an der Decke der Reinigungskammer 102 und/oder über die Düsen 110, 112, die im rückwandseitigen Bereich der Reinigungskammer 102 angeordnet sind, in diese eingespritzt werden. Die Düsen 108, 110, 112 können auch an den Seitenwänden oder an der der Reinigungskammer 102 zuweisenden Seite der der Reinigungskammer 102 verschließenden Gerätetür 114 angeordnet sein.

Über eine analog zur Steuerung 50 der Einkammer-Geschirrspülmaschine 10 aufgebaute Steuerung 50 lässt sich der Programmablauf innerhalb des Reinigungs- und Desinfektionsautomaten 100 steuern. In der Steuerung 50 werden die Wärmeäquivalenzwerte gemäß des NSF 3-Standards oder gemäß, der EN ISO 15883 implementiert. Der in der Reinigungskammer 102 in den beispielhaft dargestellten Einbaupositionen 72 beziehungsweise 74 dauerhaft eingebaute Temperatursensor 44 erfasst während des Programmablaufs innerhalb des Reinigungs- und Desinfektionsautomaten 100 die sich jeweils einstellende Temperatur. Gleichzeitig wird über die Steuerung 50 die Einwirkzeit bei der jeweiligen Temperatur ermittelt. Daraus errechnet sich die Steuerung 50, die dementsprechenden Wärmeäquivalenzwerte. Die errechneten Wärmeäquivalenzwerte werden nachfolgend mit in der Steuerung 50 hinterlegten Werten für Wärmeäquivalente entsprechend den Normen NSF Standard 3 oder EN ISO 15883 verglichen, um die sich jeweils in der Reinigungskammer 102 einstellende Hygienewirkung des Reinigungs- und Desinfektionsautomaten 100 zu beurteilen. Die sich während des Betriebs des Reinigungs- und Desinfektionsautomaten 100 einstellenden Wärmeäquivalenzwerte können über ein am Reinigungs- und Desinfektionsautomaten 100 angeordneten Display dem Bediener oder dem Betreiber des Reinigungs- und Desinfektionsautomaten 100 kontinuierlich angezeigt werden. Analog zur Darstellung gemäß Figur 1 lässt sich der Verriegelungsmechanismus 88, der der Gerätetür 114 zugeordnet ist und der als Elektromagnet ausgestaltet sein kann, bei Erreichen der eine ausreichende Hygienewirkung sicherstellenden Wärmeäquivalentwerte über die dem Reinigungs- und Desinfektionsautomaten zugeordnete Steuerung 50 entriegeln. Das Erreichen der vorgeschriebenen Wärmeäquivalente innerhalb der Reinigungskammer 102 des Reinigungs- und Desinfektionsautomaten 100 kann auch über ein akkustisches oder ein optisches Signal angezeigt werden.

Nach Erreichen der vorgeschriebenen Wärmeäquivalentwerte innerhalb der Reinigungskammer 102 des Reinigungs- und Desinfektionsautomaten 100 ist eine Entnahme hygienisch gereinigten Reinigungsgutes aus der Reinigungskammer 102 ohne Weiteres möglich.

### Bezugszeichenliste

- 10: Einkammer-Geschirrspülmaschine
- 12: Korb
- 14: Spülgut
- 16: Tank für Spüllauge
- 18: Heizelement für Spüllauge
- 20: Umwälzpumpe für Spüllauge
- 22: Spülsystem für Spüllauge
- 24: Düsen für Spüllauge
- 26: Frischwasser-Boiler
- 28: Füllventil
- 30: Frischwasser
- 32: Heizelement für Frischwassertank
- 34: Bedeckungsniveau
- 36: Entlüftungsleitung
- 38: Ansaugstelle
- 40: Spülsystem für Frischwasser
- 42: Düsen für Frischwasser
- 44: Temperatursensor
- 46: Gerätetür
- 48: Ladeöffnung
- 50: Steuerung
- 52: Mikroprozessor (CPU)
- 54: Datenspeicher
- 56: Hauptsteuerleitung
- 58: Messdatenerfassungseinheit
- 60: Leistungsregler für Umwälzpumpe 20
- 62: elektrische Energieversorgung Umwälzpumpe 20
- 64: Frischwasserpumpe
- 66: elektrische Energieversorgung Frischwasserpumpe
- 68: Leistungsregler für Frischwasserpumpe 64
- 70: Spülkammer
- 72: erste Temperatursensor-Position
- 74: zweite Temperatursensor-Position
- 76: Frischwasserleitung
- 78: Spüllaugenleitung
- 80: Energieversorgung für Heizelement 18
- 82: Leistungsregler für Heizelement
- 84: Energieversorgung für Heizelement 32
- 86: Leistungsregler für Heizelement 32
- 88: Verriegelungseinrichtung
- 90: Display

- 100: Reinigungs- und Desinfektionsautomat
- 102: Reinigungskammer
- 104: Ablauf
- 106: Siphonbogen
- 108: erste Düse
- 110: zweite Düse
- 112: dritte Düse
- 114: Gerätetür

- 124: Kaltwasser-/Warmwasserzulauf
- 125: Leitungsabschnitt
- 126: Zuleitung zur Reinigungskammer
- 127: Rückschlagventil
- 128: Wasserpumpe
- 130: Wasser-/Dampf-Einheit
- 136: Boden
- 138: Wasserbehälter
- 140: Wasserpegel im Wasserbehälter
- 142: Dampferzeuger
- 144: Wasserpegel im Dampferzeuger
- 146: Trennwand
- 148: Überströmleitung
- 150: Heizeinrichtung
- 158: Öffnungs-/Schließrichtung
- 160: Sicherheitsüberlauf

## Patentansprüche

1. Verfahren zur Beurteilung und Sicherstellung der thermischen Hygienewirkung auf Spülgut (14), welches in einer Spülkammer (70) einer Geschirrspülmaschine (10) aufgenommen ist oder auf Reinigungsgut, welches in einer Reinigungskammer (102) eines Reinigungs- und Desinfektionsautomaten (100) aufgenommen ist, wobei ein Temperatursensor (44) in die Spülkammer (70) oder in die Reinigungskammer (102) dauerhaft eingebaut ist, **dadurch gekennzeichnet, dass** über den Temperatursensor (44) und eine Steuerung (50) die thermische Hygienewirkung die auf das Spülgut (14) oder auf das Reinigungsgut einwirkende Temperatur und Einwirkzeit erfasst werden und daraus entsprechende Wärmeäquivalentwerte errechnet werden, um die thermische Hygienewirkung zu beurteilen.

2. Verfahren gemäß Anspruch 1, **dadurch gekennzeichnet, dass** die Beurteilung der thermischen Hygienewirkung durch die Steuerung (50) anhand der NSF Standard 3 Vorschrift vorgenommen wird, deren Werte für Wärmeäquivalente HUE in einem Datenspeicher (54) der Steuerung (50) abgelegt sind.

3. Verfahren gemäß Anspruch 1, **dadurch gekennzeichnet, dass** die Beurteilung der thermischen Hygienewirkung durch die Steuerung (50) der Einkammer-Geschirrspülmaschine (10) oder des Reinigungs- und Desinfektionsautomaten (100) entsprechend der A₀-Werte der EN ISO 15883, Anhang A, vorgenommen wird.

4. Verfahren gemäß Anspruch 1, **dadurch gekennzeichnet, dass** ein innerhalb der Spülkammer (70) oder innerhalb der Reinigungskammer (102) ablaufender Programmschritt zur Sicherstellung der thermischen Hygienewirkung nach Erreichen eines voreingestellten, mindest zu erreichenden Wertes von Wärmeäquivalenten gemäß NSF 3 Standard oder EN ISO 15883 beendet wird.

5. Verfahren gemäß Anspruch 4, **dadurch gekennzeichnet, dass** das Ende des Programmschritts nach Anspruch 4 durch ein optisches Signal angezeigt wird.

6. Verfahren gemäß Anspruch 4, **dadurch gekennzeichnet, dass** das Ende des Programmschritts nach Anspruch 4 durch ein akustisches Signal angezeigt wird.

7. Verfahren gemäß Anspruch 4, **dadurch gekennzeichnet, dass** bei Programmstart eines Programmablaufs eine Gerätetür (46, 114) zur Spülkammer (70) des Einkammer-Geschirrspülautomaten (10) oder zur Reinigungskammer (102) des Reinigungs- und Desinfektionsautomaten (100) verriegelt wird und nach Erreichen eines in der Steuerung (50) voreingestellten Wertes für die Wärmeäquivalente wieder freigegeben wird.

8. Verfahren gemäß der Ansprüche 1 bis 3, **dadurch gekennzeichnet, dass** während des Programmablaufs innerhalb der Spülkammer (70) oder der Reinigungskammer (102) der jeweils aktuell erreichte Wert für die Wärmeäquivalente über eine optische Anzeige (90) dem Bediener angezeigt wird.

9. Verfahren gemäß der Ansprüche 1 bis 3, **dadurch gekennzeichnet, dass** während des Programmablaufs innerhalb der Spülkammer (70) oder der Reinigungskammer (102) der jeweils aktuell erreichte A₀-Wert über ein akustisches Signal dem Bediener des Einkammer-Geschirrspülautomaten (10) oder des Reinigungs- und Desinfektionsautomaten (100) angezeigt wird.

10. Einkammer-Geschirrspülautomat (10) mit einer Steuerung (50) und einem mit dieser gekoppelten Temperatursensor (44) zur Durchführung des Verfahrens gemäß einem oder mehrerer der vorangehenden Ansprüche 1 bis 9, **dadurch gekennzeichnet, dass** in der Spülkammer (70) des Einkammer-Geschirrspülautomaten (10) der Temperatursensor (44) in einer ersten Temperatursensorposition (72) oder einer zweiten Temperatursensorposition (74) dauerhaft installiert ist.

11. Einkammer-Geschirrspülautomat gemäß Anspruch 10, **dadurch gekennzeichnet, dass** der Temperatursensor (44) im oberen Bereich der Spülkammer (70) aufgenommen ist.

12. Einkammer-Geschirrspülautomat gemäß Anspruch 10, **dadurch gekennzeichnet, dass** der Temperatursensor (44) an der Rückseite der Spülkammer (70), der Gerätetür (46) gegenüberliegend angeordnet ist.

13. Reinigungs- und Desinfektionsautomat (100) mit einer Steuerung (50) und einem mit dieser gekoppelten Temperatursensor (44) zur Durchführung des Verfahrens gemäß einem oder mehrerer der Ansprüche 1 bis 9, **dadurch gekennzeichnet, dass** in der Reinigungskammer (102) des Reinigungs- und Desinfektionsautomaten (100) der Temperatursensor (44) in einer ersten Temperatursensorposition (42) oder einer zweiten Temperatursensorposition (74) dauerhaft installiert ist.

14. Reinigungs- und Desinfektionsautomat (100) gemäß Anspruch 13, **dadurch gekennzeichnet, dass** der Temperatursensor (44) im oberen Bereich der Reinigungskammer (102) aufgenommen ist.

15. Reinigungs- und Desinfektionsautomat (100) gemäß Anspruch 13, **dadurch gekennzeichnet, dass** der Temperatursensor (44) an der Rückseite der Reinigungskammer (102), der Gerätetür (114) gegenüberliegend angeordnet ist.

## Claims

1. Method for assessing and guaranteeing the thermal hygiene effect on items (14) to be washed which are accommodated in a washing chamber (70) of a dishwasher (10) or on items to be cleaned which are accommodated in a cleaning chamber (102) of a cleaning and disinfecting machine (100), with a temperature sensor (44) being permanently installed in the washing chamber (70) or in the cleaning chamber (102), **characterized in that** the thermal hygiene effect is detected by means of the temperature sensor (44) and a control system (50), with the temperature and action time acting on the items (14) to be washed or on the items to be cleaned being detected, and with corresponding heat equivalent values being calculated in order to assess the thermal hygiene effect.

2. Method according to Claim 1, **characterized in that** the thermal hygiene effect is assessed by the control system (50) on the basis of the NSF standard 3 regulation whose values for heat equivalents HUE are stored in a data memory (54) of the control system (50).

3. Method according to Claim 1, **characterized in that** the thermal hygiene effect is assessed by the control system (50) of the single-chamber dishwasher (10) or the cleaning and disinfecting machine (100) in accordance with the A₀ values of EN ISO 15883, annex A.

4. Method according to Claim 1, **characterized in that** a program step which runs within the washing chamber (70) or within the cleaning chamber (102) in order to guarantee the thermal hygiene effect is terminated after a preset value of heat equivalents, which has to be reached at the minimum, according to NSF 3 standard or EN ISO 15883 is reached.

5. Method according to Claim 4, **characterized in that** the end of the program step according to Claim 4 is indicated by a visual signal.

6. Method according to Claim 4, **characterized in that** the end of the program step according to Claim 4 is indicated by an acoustic signal.

7. Method according to Claim 4, **characterized in that** an appliance door (46, 114) to the washing chamber (70) of the single-chamber dishwashing machine (10) or to the cleaning chamber (102) of the cleaning and disinfecting machine (100) is locked when a program run starts and is released again after a value for the heat equivalents which is preset in the control system (50) is reached.

8. Method according to Claims 1 to 3, **characterized in that** the value for the heat equivalents which is actually reached in each case is indicated to the user by means of a visual display (90) during the program run within the washing chamber (70) or the cleaning chamber (102).

9. Method according to Claims 1 to 3, **characterized in that** the A₀ value which is actually reached in each case is indicated to the user of the single-chamber dishwashing machine (10) or the cleaning and disinfecting machine (100) by means of an acoustic signal during the program run within the washing chamber (70) or the cleaning chamber (102).

10. Single-chamber dishwashing machine (10) having a control system (50) and a temperature sensor (44) coupled to this control system for carrying out the method according to one or more of the preceding Claims 1 to 9, **characterized in that** the temperature sensor (44) is permanently installed in a first temperature-sensor position (72) or a second temperature-sensor position (74) in the washing chamber (70) of the single-chamber dishwashing machine (10).

11. Single-chamber dishwashing machine according to Claim 10, **characterized in that** the temperature sensor (44) is accommodated in the upper region of the washing chamber (70).

12. Single-chamber dishwashing machine according to Claim 10, **characterized in that** the temperature sensor (44) is arranged opposite the appliance door (46), on the rear face of the washing chamber (70).

13. Cleaning and disinfecting machine (100) having a control system (50) and a temperature sensor (44) coupled to this control system for carrying out the method according to one or more of Claims 1 to 9, **characterized in that** the temperature sensor (44) is permanently installed in a first temperature-sensor position (42) or a second temperature-sensor position (74) in the cleaning chamber (102) of the cleaning and disinfecting machine (100).

14. Cleaning and disinfecting machine (100) according to Claim 13, **characterized in that** the temperature sensor (44) is accommodated in the upper region of the cleaning chamber (102).

15. Cleaning and disinfecting machine (100) according to Claim 13, **characterized in that** the temperature sensor (44) is arranged opposite the appliance door (114), on the rear face of the cleaning chamber (102).

## Revendications

1. Procédé pour évaluer et garantir l'effet hygiénique thermique sur un article à laver (14) logé dans une chambre de lavage (70) d'un lave-vaisselle (10) ou sur un article à nettoyer logé dans une chambre de nettoyage (102) d'un automate de nettoyage et de désinfection (100), un capteur de température (44) étant monté de manière permanente dans la chambre de lavage (70) ou dans la chambre de nettoyage (102), **caractérisé en ce que** l'effet hygiénique thermique, la température agissant sur l'article à laver (14) ou sur l'article à nettoyer ainsi que le temps d'action sont détectés par l'intermédiaire du capteur de température (44) et d'un dispositif de commande (50), et des valeurs d'équivalents de chaleur correspondantes sont calculées à partir de ces données pour évaluer l'effet hygiénique thermique.

2. Procédé selon la revendication 1, **caractérisé en ce que** l'évaluation de l'effet hygiénique thermique par le dispositif de commande (50) est effectuée à l'aide de la réglementation NSF standard 3, dont les valeurs pour des équivalents de chaleur HUE sont stockées dans une mémoire de données (54) du dispositif de commande (50).

3. Procédé selon la revendication 1, **caractérisé en ce que** l'évaluation de l'effet hygiénique thermique par le dispositif de commande (50) du lave-vaisselle (10) à une seule chambre ou de l'automate de nettoyage et de désinfection (100) est effectuée selon les valeurs A₀ de la norme EN ISO 15883, Annexe A.

4. Procédé selon la revendication 1, **caractérisé en ce qu'**une étape du programme se déroulant à l'intérieur de la chambre de lavage (70) ou à l'intérieur de la chambre de nettoyage (102), destinée à garantir l'effet hygiénique thermique, est arrêtée après avoir atteint une valeur préréglée, à atteindre au minimum, d'équivalents de chaleur selon le standard NSF 3 ou EN ISO 15883.

5. Procédé selon la revendication 4, **caractérisé en ce que** la fin de l'étape du programme selon la revendication 4 est signalée par un signal optique.

6. Procédé selon la revendication 4, **caractérisé en ce que** la fin de l'étape du programme selon la revendication 4 est signalée par un signal acoustique.

7. Procédé selon la revendication 4, **caractérisé en ce qu'**au lancement du déroulement d'un programme, une porte (46, 114) de l'appareil donnant accès à la chambre de lavage (70) du lave-vaisselle (10) à une seule chambre ou à la chambre de nettoyage (102) de l'automate de nettoyage et de désinfection (100) est verrouillée et, après avoir atteint une valeur préréglée dans le dispositif de commande (50) pour les équivalents de chaleur, est à nouveau déverrouillée.

8. Procédé selon les revendications 1 à 3, **caractérisé en ce qu'**au cours du déroulement du programme à l'intérieur de la chambre de lavage (70) ou de la chambre de nettoyage (102), la valeur actuellement atteinte pour les équivalents de chaleur est signalée par un affichage optique (90) à l'utilisateur.

9. Procédé selon les revendications 1 à 3, **caractérisé en ce qu'**au cours du déroulement du programme à l'intérieur de la chambre de lavage (70) ou de la chambre de nettoyage (102), la valeur A₀ actuellement atteinte est signalée par un signal acoustique à l'utilisateur du lave-vaisselle (10) à une seule chambre ou de l'automate de nettoyage et de désinfection (100).

10. Lave-vaisselle (10) à une seule chambre comprenant un dispositif de commande (50) et, couplé à ce dernier, un capteur de température (44) pour la mise en oeuvre du procédé selon une ou plusieurs des revendications précédentes 1 à 9, **caractérisé en ce que** le capteur de température (44) est monté dans la chambre de lavage (70) dudit lave-vaisselle (10) à une seule chambre, de manière permanente, dans une première position (72) du capteur de température ou dans une deuxième position (74) du capteur de température.

11. Lave-vaisselle à une seule chambre selon la revendication 10, **caractérisé en ce que** le capteur de température (44) est logé dans la partie supérieure de la chambre de lavage (70).

12. Lave-vaisselle à une seule chambre selon la revendication 10, **caractérisé en ce que** le capteur de température (44) est agencé sur la face arrière de la chambre de lavage (70), à l'opposé de la porte (46) de l'appareil.

13. Automate de nettoyage et de désinfection (100) comprenant un dispositif de commande (50) et, couplé à ce dernier, un capteur de température (44) pour la mise en ceuvre du procédé selon une ou plusieurs des revendications 1 à 9, **caractérisé en ce que** le capteur de température (44) est monté dans la chambre de nettoyage (102) de l'automate de nettoyage et de désinfection (100), de manière permanente, dans une première position (42) du capteur de température ou dans une deuxième position (74) du capteur de température.

14. Automate de nettoyage et de désinfection (100) selon la revendication 13, **caractérisé en ce que** le capteur de température (44) est logé dans la partie supérieure de la chambre de nettoyage (102).

15. Automate de nettoyage et de désinfection (100) selon la revendication 13, **caractérisé en ce que** le capteur de température (44) est agencé sur la face arrière de la chambre de nettoyage (102), à l'opposé de la porte (114) de l'appareil.
